Europäisches Patentamt

European Patent Office

Office européen des brevets

(19)

(11)   EP 0 999 183 B1

(12)   FASCICULE DE BREVET EUROPEEN

(45) Date de publication et mention
de la délivrance du brevet:
**18.06.2003 Bulletin 2003/25**

(51) Int Cl.⁷: **C01B 39/48**, B01D 15/00,
B01J 29/70, C10G 45/64,
C10G 35/095, C10G 49/08

(21) Numéro de dépôt: **99402633.4**

(22) Date de dépôt: **21.10.1999**

(54) **Procédé de préparation d'une zéolithe de type structural EUO a l'aide de precurseurs du structurant et son utilisation comme catalyseur d'isomerisation des AC8**

Verfahren zur Herstellung eines Zeoliths des EUO-Typs mittels Vorläufern des Strukturbildners und dessen Verwendung als Isomerisierungskatalysator von Aromaten mit acht Kohlenstoffatomen

Process for the preparation of EUO-type zeolite using structuring agent precursors and use thereof as catalyst for isomerizing eight carbon aromatics

(84) Etats contractants désignés:
**DE DK GB NL**

(30) Priorité: **02.11.1998 FR 9813773**
**23.12.1998 FR 9816411**

(43) Date de publication de la demande:
**10.05.2000 Bulletin 2000/19**

(73) Titulaire: **Institut Francais du Petrole**
**92852 Rueil Malmaison Cedex (FR)**

(72) Inventeurs:
• **Rouleau, Loic**
**69600 Oullins (FR)**
• **Lacombe, Sylvie**
**92500 Rueil-Malmaison (FR)**
• **Alario, Fabio**
**92200 Neuilly sur Seine (FR)**
• **Merlen, Elisabeth**
**92500 Reuil-Malmaison (FR)**
• **Kolenda, Frédéric**
**69630 Chaponost (FR)**

(56) Documents cités:
EP-A- 0 042 226      EP-A- 0 051 318
EP-A- 0 463 768      WO-A-96/29284
US-A- 4 275 047

• CASCI J L ET AL: "SYNTHESIS AND
CHARACTERISATION OF ZEOLITE EU-1"
PROCEEDINGS OF THE SIXTH INTERNATIONAL
ZEOLITE CONFERENCE.;RENO, NV, USA,1984,
pages 894-904, XP002108428 1984 Engl,
Butterworths & Co, Guildford, Engl

**Description**

Domaine technique

La présente invention concerne un nouveau procédé de préparation des zéolithes de type structural EUO. Les zéolithes de type structural EUO synthétisées selon le procédé de la présente invention comprennent les zéolithes EU-1 et TPZ-3. Ces zéolithes ont généralement la formule suivante sous forme anhydre 0 à 20 $R_2O$ : 0-10 $T_2O_3$ : 100 $XO_2$ où R représente un cation monovalent ou 1/n d'un cation de valence n, T représente au moins un élément choisi parmi l'aluminium, le fer, le gallium, le bore, le titane, le vanadium, le zirconium, le molybdène, l'arsenic, l'antimoine, le chrome et le manganèse, X représente le silicium et/ou le germanium.
Les zéolithes de type structural EUO telles les zéolithes EU-1 et TPZ-3 sont généralement synthétisées par mélange en milieu aqueux d'au moins une source de silice et/ou de germanium et d'au moins une source d'au moins un élément choisi parmi l'aluminium, le fer, le gallium, le bore, le titane, le vanadium, le zirconium, le molybdène, l'arsenic, l'antimoine, le chrome et le manganèse en présence d'un composé organique comprenant un dérivé alkylé de polyméthylène $\alpha$-$\omega$ diammonium, jouant le rôle de structurant Le mélange est généralement maintenu à une certaine température jusqu'à la cristallisation de la zéolithe.
La présente invention concerne également un catalyseur à base de zéolithe de structure EUO, ladite zéolithe étant obtenue selon le nouveau mode de synthèse décrit ci-dessus, ainsi que le procédé de préparation dudit catalyseur. L'invention concerne également un procédé d'isomérisation des composés aromatiques à 8 atomes de carbone encore appelés « coupes C8 aromatiques » en présence de ce catalyseur à base de zéolithe de type structural EUO.
L'isomérisation en xylènes de l'éthylbenzène nécessite la présence d'un métal du groupe VIII. Les formulations optimisées à base de mordénite et d'un métal du groupe VIII conduisent à des catalyseurs avec lesquels les réactions parasites restent non négligeables. Par exemple, on peut citer l'ouverture de cycles naphténiques suivie ou non de craquage ou encore les réactions de dismutation et de transalkylation des aromatiques en C8 qui conduisent à la formation d'aromatiques non recherchés. Il est donc particulièrement intéressant de trouver de nouveaux catalyseurs plus sélectifs.

Art antérieur

La zéolithe EU-1 de type structural EUO, déjà décrite dans l'art antérieur, présente un réseau microporeux monodimensionnel, dont le diamètre des pores est de 4,1 x 5,7Å (1 Å = 1 Angström = $1.10^{-10}$ m) (« Atias of Zeolites Structure types », W.M. Meier et D.H. Oison, 4$^{ème}$ Edition, 1996). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. Le mode de synthèse de la zéolithe EU-1 et ses caractéristiques physico-chimiques ont été décrits dans le brevet EP- 42 226. Le mode de synthèse comprend le mélange d'un oxyde de silicium et/ou de germanium et d'un oxyde d'au moins un élément choisi parmi l'aluminium, le fer, le gallium et le bore, en présence d'un structurant comprenant au moins un dérivé alkylé de polyméthylène $\alpha$-$\omega$ diammonium de formule $R_1R_2R_3 N^+ (CH_2)_n N^+ R_4R_5R_6$, les produits de dégradation dudit dérivé ou les précurseurs dudit dérivé. Les précurseurs du dérivé alkylé sont la diamine apparentée conjointement avec des alcools ou des halogénures d'alkyle.
La demande de brevet EP 51 318 traite de la zéolithe TPZ-3, qui présente d'après l' « Atlas of Zeolites Structure types », W.M. Meier et D.H. Olson, 4$^{ème}$ Edition, 1996, le même type structural EUO que la zéolithe EU-1. La préparation de la zéolithe comprend le mélange d'un composé de métal alcalin soluble, un composé de 1,6-N,N,N,N', N',N'-hexaméthylhexaméthylènediammonium, un composé capable de donner de la silice et un composé capable de donner de l'alumine, à une température supérieure à 80°C.
La demande de brevet internationale WO 96/29284 divulgue une méthode de préparation de zéolithes de différents types structuraux comprenant le mélange en milieu aqueux d'un oxyde de silicium et/ou de germanium, d'un oxyde d'aluminium, de fer, de gallium, de bore et/ou de titane, d'un métal alcalin, d'un composé aminé contenant de un à huit atomes de carbone et d'un structurant organique. Pour la synthèse d'une zéolithe de type structural EUO, ce document enseigne notamment l'utilisation des sels de 1,6,N,N,N,N',N',N'-hexaméthylhexaméthylène diammonium ou d'un composé dérivé du sel 4-benzyl-N,N-diméthylpiperidinium en tant que structurant organique. Le composé aminé utilisé pour la synthèse des zéolithes est généralement une amine aliphatique ou une amine cycloaliphatique.

Résumé de l'invention

La présente invention concerne un nouveau procédé de préparation d'un matériau zéolithique de type structural EUO en présence d'au moins deux précurseurs d'un dérivé alkylé de polyméthylène $\alpha$-$\omega$ diammonium jouant le rôle de structurant. La présente invention concerne également l'utilisation de ladite zéolithe dans un catalyseur comprenant en outre au moins un élément du groupe VIII de la classification périodique des éléments et au moins un liant.

Ledit catalyseur peut être utilisé dans un procédé d'isomérisation des composés aromatiques à 8 atomes de carbone.

Intérêt de l'invention

Le procédé selon l'invention permet de réduire le temps de cristallisation de la zéolithe après formation du mélange, ce qui procure un gain de coût. De plus, l'utilisation de précurseurs du structurant selon l'invention permet d'accroître la sécurité lors de la synthèse de la zéolithe, lesdits précurseurs étant moins dangereux que le structurant lui-même ou que les précurseurs de l'art antérieur, et permet également de réduire le coût des réactifs, lesdits précurseurs étant moins onéreux que le structurant lui-même ou que les précurseurs de l'art antérieur.

Ainsi la demanderesse a découvert de manière surprenante que la synthèse d'une zéolithe caractérisée par l'utilisation d'au moins deux précurseurs spécifiques du structurant permettait notamment d'obtenir les avantages cités ci-dessus, c'est-à-dire un gain de temps, de sécurité et de coût des réactifs.

Description de l'invention

L'invention concerne un procédé de synthèse d'un matériau zéolithique de type structural EUO comprenant le mélange en milieu aqueux d'au moins une source d'au moins un élément choisi parmi le silicium et le germanium et d'au moins une source d'au moins un élément T choisi parmi l'aluminium, le fer, le gallium, le bore, le titane, le vanadium, le zirconium, le molybdène, l'arsenic, l'antimoine, le chrome et le manganèse, en présence d'au moins deux précurseurs d'un dérivé alkylé de polyméthylène $\alpha$-$\omega$ diammonium jouant le rôle de structurant. Le mélange est généralement maintenu à une certaine température jusqu'à la cristallisation de la zéolithe. L'invention est caractérisée en ce qu'on utilise au moins deux précurseur du dérivé alkylé de polyméthylène $\alpha$-$\omega$ diammonium l'un étant choisi parmi les monoamines et l'autre étant du type F-R-F'où F et F' vont des groupements partants identique ou différents.

Le dérivé alkylé de polyméthylène $\alpha$-$\omega$ diammonium jouant le rôle de structurant a la formule suivante :

$$R_1R_2R_3 \ N^+ \ (CH_2)_n \ N^+ \ R_4R_5R_6$$

n étant compris entre 3 et 14 et $R_1$ à $R_6$, identiques ou différents, pouvant représenter des radicaux alkyles ou hydroxyalkyles ayant de 1 à 8 atomes de carbone, jusqu'à cinq radicaux $R_1$ à $R_6$ pouvant être de l'hydrogène.

En plus du (des) précurseur(s) du structurant choisi(s) parmi les monoamines selon le procédé de la présente invention, le(s) autre(s) groupement(s) du structurant est (sont) généralement introduit(s) à l'aide de tout précurseur approprié pour obtenir un ammonium quaternaire. Ces précurseurs sont du type F-R-F', où F et F' sont des groupements partants identiques ou différents tels que par exemple une fonction alcool ou un halogénure. A titre d'exemple on choisit en outre comme précurseur au moins un composé choisi parmi les alcanediols et les dihalogénures d'alcane.

Les précurseurs du structurant selon l'invention et les autres précurseurs peuvent être préchauffés ensemble dans le récipient de réaction ou peuvent être mélangés tels quels avec les autres réactifs. Les précurseurs peuvent être introduits à n'importe quel moment de la préparation de la zéolithe.

De préférence, on introduit les précurseurs du structurant en solution avant l'addition des autres réactifs nécessaires pour la synthèse de la zéolithe.

Dans une mise en oeuvre particulière, il peut être avantageux d'ajouter au milieu réactionnel des germes S d'au moins une zéolithe. On peut ajouter des germes ayant le type structural de la zéolithe EUO ou le type structural d'autres zéolithes accessibles et peu onéreuses comme par exemple les zéolithes de type structural LTA, FAU, MOR, MFI. Ces germes peuvent accélérer la cristallisation de la zéolithe EUO à partir du mélange réactionnel. Les germes peuvent être introduits à tout moment de la synthèse de la zéolithe. De préférence, dans le cas éventuel où la zéolithe EUO est synthétisée à l'aide de germes, lesdits germes sont ajoutés après homogénéisation au moins en partie du mélange contenant les autres réactifs.

Dans une autre mise en oeuvre particulière, indépendante ou non de la mise en oeuvre précédente, il peut être avantageux d'ajouter au milieu réactionnel au moins un sel P de métal alcalin ou d'ammonium. On peut citer par exemple des radicaux acides forts tels que du bromure, du chlorure, de l'iodure, du sulfate, du phosphate ou du nitrate, ou des radicaux acides faibles tels que les radicaux acides organiques, par exemple du citrate ou de l'acétate. Ce sel peut accélérer la cristallisation des zéolithes EUO à partir du mélange réactionnel.

Le mélange aqueux réactionnel a généralement la composition molaire suivante, exprimée sous la forme d'oxyde :

| | |
|---|---|
| $XO_2/T_2O_3$ (mol/mol) | au moins 10 |
| $OH^-/XO_2$ (mol/mol) | 0,002 à 2,0 |

(suite)

| Q/XO$_2$ (mol/mol) | 0,002 à 2,0 |
|---|---|
| Q/(M$^+$ + Q) (mol/mol) | 0,1 à 1,0 |
| H$_2$O/XO$_2$ (mol/mol) | 1 à 500 |
| P/XO$_2$ (mol/mol) | 0 à 5 |
| S/XO$_2$ (g/g) | 0 à 0,1 |

de manière préférée, le mélange réactionnel a la composition suivante, exprimée sous forme d'oxydes

| XO$_2$/T$_2$O$_3$ (mol/mol) | au moins 12 |
|---|---|
| OH$^-$/XO$_2$ (mol/mol) | 0,005 à 1,5 |
| Q/XO$_2$ (mol/mol) | 0,005 à 1,5 |
| Q/(M$^+$ + Q) (mol/mol) | 0,1 à 1,0 |
| H$_2$O/XO$_2$ (mol/mol) | 3 à 250 |
| P/XO$_2$ (mol/mol) | 0 à 1 |
| S/XO$_2$ (g/g) | 0,0005 à 0,07 |

et, de manière encore plus préférée, le mélange réactionnel a la composition suivante, exprimée sous forme d'oxydes :

| XO$_2$/T$_2$O$_3$ (mol/mol) | au moins 15 |
|---|---|
| OH$^-$/XO$_2$ (mol/mol) | 0,01 à 1 |
| Q/XO$_2$ (mol/mol) | 0,01 à 1 |
| Q/(M$^+$ + Q) (mol/mol) | 0,1 à 1,0 |
| H$_2$O/XO$_2$ (mol/mol) | 5 à 100 |
| P/XO$_2$ (mol/mol) | 0 à 0,25 |
| S/XO$_2$ (g/g) | 0,001 à 0,04 |

où

X est le silicium et/ou le germanium,

T est au moins un élément choisi parmi l'aluminium, le fer, le gallium, le bore, le titane, le vanadium, le zirconium, le molybdène, l'arsenic, l'antimoine, le chrome et le manganèse

M$^+$ représente un métal alcalin ou l'ion ammonium,

Q représente le dérivé alkylé précité de polyméthylène $\alpha$-$\omega$ diammonium, introduit à l'aide des précurseurs appropriés correspondants, contenant une monoamine,

S représente les germes de zéolithe exprimé sous la forme séchée, calcinée ou échangée,

P représente le sel de métal alcalin ou d'ammonium.

M et/ou Q peuvent être présents sous forme d'hydroxydes ou de sels d'acides inorganiques ou organiques à la condition que le critère OH$^-$ / XO$_2$ soit satisfait.

L'invention est caractérisée en ce que le structurant organique comprenant un dérivé alkylé de polyméthylène $\alpha$-$\omega$ diammonium est introduit à l'aide d'au moins deux précurseurs l'un d'entre eux étant choisi parmi les monoamines. On entend par monoamine tout composé organique ayant une fonction amine. De manière préférée les précurseurs selon l'invention sont choisis parmi les alkylamines ayant de 1 à 18 atomes de carbone par molécule, et de préférence ayant de 1 à 8 atomes de carbone par molécule. Les alkylamines peuvent être primaires, secondaires ou tertiaires. Plus particulièrement les précurseurs sont choisis parmi les trialkylamines.

Les précurseurs préférés de départ sont, entre autres, ceux qui conduisent aux dérivés alkylés de polyméthylène $\alpha$-$\omega$ diammonium préférés, préférentiellement aux dérivés alkylés d'hexaméthylènediammonium et spécialement les dérivés méthylés d'hexaméthylènediammonium et encore plus préférentiellement les sels de 1,6-N,N,N,N', N',N'-hexaméthylhexaméthylènediammonium ayant la formule (CH$_3$)$_3$ N$^+$ (CH$_2$)$_6$ N$^+$ (CH$_3$)$_3$, par exemple l'halogénure, l'hydroxyde, le sulfate, le silicate, l'aluminate. Par exemple, de manière préférée, un des précurseurs selon l'invention est choisi parmi les monoamines est la triméthylamine et l'autre précurseur est le dibromohexane.

Le métal alcalin (M$^+$) préféré est le sodium. L'élément T préféré est l'aluminium L'élément X préféré est le silicium.

La source de silicium peut être l'une quelconque de celles dont l'utilisation est normalement envisagée pour la synthèse des zéolithes, par exemple la silice solide en poudre, l'acide silicique, la silice colloïdale ou la silice en solution. Parmi les silices en poudre utilisables, il convient de citer les silices précipitées, spécialement celles obtenues par précipitation à partir d'une solution d'un silicate de métal alcalin, comme les Zeosil ou Tixosil, produites par Rhône-Poulenc, les silices pyrogénées telles que les aerosil produites par Degussa et les Cabosil produites par Cabot et les gels de silice. Des silices colloïdales de diverses granulométries peuvent être utilisées, comme celles vendues sous les marques déposées « LUDOX » de Dupont, et « SYTON » de Monsantos. Les silices dissoutes utilisables sont notamment les verres solubles ou silicates commercialisés contenant : 0,5 à 6,0 et spécialement 2,0 à 4,0 moles de $SiO_2$ par mole d'oxyde de métal alcalin et les silicates obtenues par dissolution de silice dans un hydroxyde de métal alcalin, un hydroxyde d'ammonium quaternaire ou un mélange de ceux-ci.

La source d'aluminium est le plus avantageusement l'aluminate de sodium, mais peut être l'aluminium, un sel d'aluminium, par exemple le chlorure, le nitrate ou le sulfate, un alcoolate d'aluminium ou l'alumine elle-même qui devrait de préférence se trouver sous une forme hydratée ou hydratable comme l'alumine colloïdale, la pseudoboehmite, la boehmite, l'alumine gamma, ou les trihydrates.

On peut utiliser des mélanges des sources citées ci-dessus. Des sources combinées de silicium et d'aluminium peuvent aussi être mises en oeuvre telles que les silice-alumines amorphes ou certaines argiles.

Le mélange de réaction est habituellement mis à réagir sous la pression autogène, éventuellement avec apport d'un gaz, par exemple d'azote, à une température comprise entre 85 et 250°C jusqu'à ce qu'il se forme des cristaux de la zéolithe, ce qui peut durer de 1 minute à plusieurs mois suivant la composition des réactifs, le mode de chauffage et de mélange, la température de travail et l'agitation. L'agitation est facultative, mais préférable, parce qu'elle abrège la durée de réaction.

Au terme de la réaction, la phase solide est collectée sur un filtre et lavée et est alors prête pour les opérations suivantes comme le séchage, la calcination et l'échange d'ions.

Ainsi, afin d'obtenir la forme hydrogène de la zéolithe EUO, on peut effectuer un échange d'ions avec un acide, spécialement un acide minéral fort comme l'acide chlorhydrique, sulfurique ou nitrique, ou avec un composé d'ammonium tel que le chlorure, le sulfate ou le nitrate d'ammonium. L'échange d'ions peut être effectué par dilution en une ou plusieurs fois avec la solution d'échange d'ions. La zéolithe EUO peut être calcinée avant ou après l'échange d'ions ou entre deux étapes d'échange d'ions, de préférence avant l'échange d'ions afin d'éliminer toute substance organique absorbée, dans la mesure où l'échange d'ions s'en trouve facilité.

En règle générale, le ou les cations de la zéolithe EUO, peuvent être remplacés par un ou des cations quelconques de métaux et en particulier ceux des groupes IA, IB, IIA, IIB, IIIA et IIIB (y compris les terres rares) VIII (y compris les métaux nobles) de même que par le plomb, l'étain et le bismuth (Le tableau périodique est tel que dans "Handbook of Physic and Chemistry", 76ième édition). L'échange est exécuté au moyen de sels hydrosolubles quelconques contenant le cation approprié.

La présente invention concerne également l'utilisation de la zéolithe EUO telle que préparée selon le procédé de la présente invention comme adsorbant pour le contrôle de la pollution, comme tamis moléculaire pour la séparation et comme solide acide pour la catalyse dans les domaines du raffinage et de la pétrochimie.

Par exemple, lorsqu'elle est utilisée comme catalyseur, la zéolithe EUO synthétisée selon le procédé de la présente invention peut être associée à une matrice inorganique qui peut être inerte ou catalytiquement active et à une phase active. La matrice inorganique peut être présente simplement comme liant pour maintenir ensemble les petites particules de la zéolithe sous les différentes formes connues des catalyseurs (extrudés, billes, poudres), ou bien peut être ajoutée comme diluant pour imposer le degré de conversion dans un procédé qui progresserait sinon à une allure trop rapide conduisant à un encrassement du catalyseur en conséquence d'une formation exagérée de coke. Des diluants inorganiques typiques sont notamment des matières de support pour les catalyseurs comme la silice, les différentes formes d'alumine et les argiles kaoliniques, les bentonites, les montmorillonites, la sépiolite, l'attapulgite, la terre à foulon, les matières poreuses synthétiques comme $SiO_2$-$Al_2O_3$, $SiO_2$-$ZrO_2$, $SiO_2$-$ThO_2$, $SiO_2$-$BeO$, $SiO_2$-$TiO_2$ ou toute combinaison de ces composés.

La zéolithe de type structural EUO peut aussi être associée à au moins une autre zéolithe et jouer le rôle de phase active principale ou d'additif.

La matrice inorganique peut être un mélange de différents composés, en particulier d'une phase inerte et d'une phase inorganique.

La phase métallique est introduite sur la zéolithe seule, la matrice inorganique seule ou l'ensemble matrice inorganique-zéolithe par échange d'ions ou imprégnation avec des cations ou oxydes choisis parmi les suivants, Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Ru, Rh, Os, Ir et tout autre élément de la classification périodique des éléments.

Les compositions catalytiques comportant la zéolithe de type structural EUO peuvent trouver leur application dans les réactions d'isomérisation, de transalkylation et de dismutation, d'alkylation et de désalkylation, d'hydratation et de déshydratation, d'oligomérisation et de polymérisation, de cyclisation, d'aromatisation, de craquage et d'hydrocraquage, d'hydrogénation et de déshydrogénation, de reformage, d'oxydation, d'halogénation, de synthèses d'amines, d'hydrodésulfuration et d'hydrodénitrification, d'élimination catalytique des oxydes d'azote, de formation d'éther et de conversion d'hydrocarbures et de synthèse de composés organiques en général, lesdites réactions comprenant des hydrocarbures aliphatiques saturés et insaturés, des hydrocarbures aromatiques, des composés organiques oxygénés et des composés organiques contenant de l'azote et/ou du soufre, ainsi que des composés organiques contenant d'autres groupes fonctionnels.

La présente invention concerne plus particulièrement un catalyseur d'isomérisation des C8 aromatiques. Le catalyseur de la présente invention, mis sous forme de billes ou d'extrudés, contient :

- au moins une zéolithe de structure EUO, par exemple la zéolithe EU-1, caractérisée en ce que l'on utilise lors de sa synthèse au moins un précurseur du dérivé alkylé de polyméthylène $\alpha$-$\omega$ diammonium choisi parmi les monoamines selon la méthode décrite précédemment,
- au moins un métal du groupe VIII de la classification périodique des éléments, choisi de préférence dans le groupe constitué par le palladium et le platine et de manière encore plus préférée le platine,
- au moins un liant, de préférence l'alumine,
- éventuellement au moins un métal appartenant au groupe formé par les éléments des groupes IB, IIB, IIIA, IVA, VIB et VIIB de la classification périodique des éléments, de préférence l'étain ou l'indium,
- éventuellement du soufre,

ledit catalyseur étant caractérisé en ce qu'il est préparé selon un nouveau mode de synthèse de la zéolithe de type structural EUO comprise dans le tel que décrit ci-dessus .

Plus précisément le catalyseur préparé selon le procédé de la présente invention, mis sous forme de billes ou d'extrudés, comprend par rapport au poids de catalyseur :

- de 1 à 90 %, de préférence de 3 à 60 % et de manière encore plus préférée de 4 à 40 % en poids, d'au moins une zéolithe de structure EUO, obtenue selon le nouveau mode de synthèse, comprenant au moins un élément X choisi parmi le germanium et le silicium et au moins un élément T choisi dans le groupe formé par l'aluminium, le fer, le gallium, le bore, le titane, le vanadium, le zirconium, le molybdène, l'arsenic, l'antimoine, le chrome et le manganèse, de préférence l'aluminium et le bore, dont le rapport atomique X/T est supérieur ou égal à 5. La dite zéolithe est au moins en partie sous forme acide, c'est à dire sous forme hydrogène ($H^+$), la teneur en sodium étant telle que le rapport atomique Na/T est inférieur à 0,5, de préférence inférieur à 0,1, de manière encore plus préférée inférieur à 0,02,
- de 0,01 à 2 % et de préférence de 0,05 à 1,0 % en poids, d'au moins un métal du groupe VIII de la classification périodique des éléments, de préférence choisi dans le groupe formé par le platine et le palladium et de manière encore plus préférée le platine
- éventuellement de 0,01 à 2 % et de préférence entre 0,05 et 1,0 % en poids, d'au moins un métal du groupe formé par les groupes IB, IIB, IIIA, IVA, VIB et VIIB de la classification périodique des éléments, de préférence choisi dans le groupe formé par l'étain et l'indium,
- éventuellement du soufre dont la teneur est telle que le rapport du nombre d'atomes de soufre sur le nombre d'atomes de métal du groupe VIII déposés est compris entre 0,5 et 2 bornes incluses.
- le complément à 100 % en poids d'au moins un liant, de préférence de l'alumine.

Toute zéolithe de structure EUO connue de l'homme du métier et obtenue selon le mode de synthèse décrit dans le présent brevet convient pour le catalyseur préparé selon le procédé de la présente invention. Ainsi par exemple, la zéolithe utilisée comme base pour préparer ledit catalyseur peut être la zéolithe EU-1 brute de synthèse ayant les spécificités requises concernant le rapport X/T. On pourra procéder généralement à une calcination, puis à au moins un échange ionique dans au moins une solution de $NH_4NO_3$ de manière à obtenir une zéolithe dont la teneur en sodium résiduel est plus ou moins importante.

Le liant (ou matrice) compris dans le catalyseur préparé selon le procédé de la présente invention consiste généralement en au moins un élément choisi dans le groupe formé par les argiles, la magnésie, les alumines, les silices, l'oxyde de titane, l'oxyde de bore, la zircone, les phosphates d'aluminium, les phosphates de titane, les phosphates de zirconium et les silices alumines. On peut aussi utiliser du charbon. De préférence le liant est une alumine.

La zéolithe de type structural EUO, par exemple la zéolithe EU-1, comprise dans le catalyseur selon l'invention, est au moins en partie, de préférence pratiquement totalement, sous forme acide, c'est-à-dire sous forme hydrogène ($H^+$), la teneur en sodium étant de préférence telle que le rapport atomique Na/T est inférieur à 0,5, de préférence inférieur à 0,1, de manière encore plus préférée inférieur à 0,02.

Les métaux peuvent être introduits soit tous de la même façon soit par des techniques différentes, à tout moment de la préparation, avant ou après mise en forme et dans n'importe quel ordre. De plus, des traitements intermédiaires tels que par exemple une calcination et/ou une réduction peuvent être appliqués entre les dépôts des différents métaux.

Au moins un élément du groupe VIII est introduit dans la zéolithe ou sur le liant, de préférence sur le liant avant ou après mise en forme.

Une méthode préférée consiste à réaliser un mélange de la matrice et de la zéolithe suivie d'une mise en forme. La mise en forme est généralement suivie d'une calcination, généralement à une température comprise entre 250°C et 600°C bornes incluses. Au moins un élément du groupe VIII de la classification périodique des éléments est introduit après cette calcination, de préférence par dépôt sélectif sur le liant. Lesdits éléments sont déposés pratiquement à plus de 90 % totalement sur le liant de la manière connue de l'homme du métier par contrôle des paramètres utilisés lors dudit dépôt, comme par exemple la nature du précurseur utilisé pour effectuer ledit dépôt. Eventuellement, au moins un élément appartenant au groupe formé par les éléments des groupes IB, IIB, IIIA, IVA, VIB et VIIB est ajouté. On peut ajouter les éléments du groupe VIII et des groupes IB, IIB, IIIA, IVA, VIB et VIIB soit séparément à n'importe quelle étape de la préparation dudit catalyseur soit simultanément dans au moins une étape unitaire. Lorsqu'un élément au moins des groupes IB, IIB, IIIA, IVA, VIB et VIIB est ajouté séparément, il est préférable qu'il soit ajouté préalablement à l'élément du groupe VIII.

Au moins un élément du groupe VIII est déposé de manière préférée sur le mélange zéolithe-liant préalablement mis en forme par tout procédé connu de l'homme du métier. Un tel dépôt est par exemple effectué par la technique d'imprégnation à sec, d'imprégnation par excès ou d'échange ionique. Tous les précurseurs conviennent pour le dépôt de ces éléments. Par exemple, de préférence, on mettra en oeuvre un échange anionique avec de l'acide hexachloroplatinique et/ou de l'acide hexachloropalladique en présence d'un agent compétiteur, par exemple l'acide chlorhydrique. Avec de tels précurseurs, le métal est pratiquement à plus de 90 % déposé totalement sur le liant et il présente une bonne dispersion et une bonne répartition macroscopique à travers le grain de catalyseur ce qui constitue une méthode préférée de préparation.

Eventuellement au moins un autre métal choisi dans le groupe formé par les éléments des groupes IB, IIB, IIIA, IVA, VIB et VIIB de la classification périodique des éléments est également introduit. Toutes les techniques de dépôt connues de l'homme du métier et tous les précurseurs conviennent pour l'introduction d'au moins un métal additionnel.

Une des méthodes préférées de préparation du catalyseur, préparé selon le procédé de la présente invention, consiste à malaxer la zéolithe dans un gel humide de matrice (obtenu généralement par mélange d'au moins un acide et d'une poudre de matrice), par exemple l'alumine, pendant une durée nécessaire à l'obtention d'une bonne homogénéité de la pâte ainsi obtenue, soit par exemple pendant une dizaine de minutes, puis à passer la pâte à travers une filière pour former des extrudés, par exemple de diamètre compris entre 0,4 et 4 mm bornes incluses. Puis après séchage par exemple pendant quelques heures à environ 120°C en étuve et après calcination, par exemple pendant deux heures à environ 500°C, au moins un élément, par exemple le platine, est déposé, par exemple par échange anionique avec de l'acide hexachloroplatinique en présence d'un agent compétiteur (par exemple l'acide chlorhydrique), ledit dépôt étant suivi d'une calcination par exemple pendant environ 2 heures à environ 500°C.

Le platine est généralement introduit dans la matrice sous forme d'acide hexachloroplatinique, mais pour tout métal noble peuvent être également utilisés des composés ammoniaqués ou des composés tels que par exemple le chloroplatinate d'ammonium, le dichlorure de platine dicarbonyle, l'acide hexahydroxyplatinique, le chlorure de palladium, le nitrate de palladium.

L'emploi dans la présente invention d'au moins un métal noble de la famille du platine peut à titre d'exemple se faire grâce à l'utilisation de composés ammoniaqués. Dans ce cas, le métal noble sera déposé dans la zéolithe.

Dans le cas du platine, on peut citer par exemple les sels de platine II tétramines de formule $Pt(NH_3)_4X_2$, les sels de platine IV hexamines de formule $Pt(NH_3)_6X_4$; les sels de platine IV halogénopentamines de formule $(PtX(NH_3)_5)X_3$ ; les sels de platine N tétrahalogénodiamines de formule $PtX_4(NH_3)_2$ ; les complexes de platine avec les halogènes-polycétones et les composés halogénés de formule $H(Pt(acac)_2X)$ ; X étant un halogène choisi dans le groupe formé par le chlore, le fluor, le brome et l'iode , et de préférence X étant le chlore, et acac représentant le groupe $C_5H_7O_2$ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Le métal additionnel, éventuellement introduit en plus, choisi dans le groupe formé par les éléments des groupes IB, IIB, IIIA, IVA, VIB et VIIB, peut être introduit par l'intermédiaire de composés tels que par exemple les chlorures, les bromures et les nitrates, les alkyls d'éléments des groupes IB, IIB, IIIA, IVA, VIB et VIIB, soit par exemple l'étain et l'indium, les alkyl étain, le nitrate et le chlorure d'indium.

Ce métal peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés du métal, on peut citer en particulier le tétrabutylétain dans le cas de l'étain, et le triphénylindium dans le cas de l'indium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

On peut aussi envisager d'introduire au moins un métal choisi dans le groupe formé par les éléments des groupes IB, IIB, IIIA, IVA, VIB et VIIB. Ce métal additionnel peut être éventuellement introduit à tout moment de la préparation, de préférence préalablement au dépôt d'un ou plusieurs métaux du groupe VIII. Si ce métal est introduit avant le métal noble, le composé du métal utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal par exemple le tétrabutylétain. Dans ce cas, avant de procéder à l'introduction d'au moins un métal noble, on procède à une calcination sous air.

La mise en forme du catalyseur selon l'invention est généralement telle que le catalyseur est de préférence sous forme d'extrudés ou de billes en vue de son utilisation.

La préparation du catalyseur se termine généralement par une calcination, habituellement à une température comprise entre environ 250°C et 600°C bornes incluses, pour une durée d'environ 0,5 à 10 heures, de préférence précédée d'un séchage, par exemple à l'étuve, à une température comprise entre la température ambiante et 250°C, de préférence entre 40 et 200°C. Ladite étape de séchage est de préférence menée pendant la montée en température nécessaire pour effectuer ladite calcination.

Dans le cas où le catalyseur de la présente invention contient du soufre, le soufre est introduit sur le catalyseur mis en forme, calciné, contenant le ou les métaux cités précédemment, soit *in-situ* avant la réaction catalytique, soit *ex-situ*. La sulfuration éventuelle intervient après la réduction. Dans le cas d'une sulfuration in situ, la réduction, si le catalyseur n'a pas été préalablement réduit, intervient avant la sulfuration. Dans le cas d'une sulfuration ex-situ, on effectue la réduction puis la sulfuration. La sulfuration s'effectue en présence d'hydrogène en utilisant tout agent sulfurant bien connu de l'homme de métier, tel que par exemple le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal soit de 1,5. Le catalyseur est ensuite maintenu pendant environ 3 heures à environ 400°C sous débit d'hydrogène avant l'injection de la charge.

La présente invention concerne également un procédé d'isomérisation des coupes C8 aromatiques constituées d'un mélange de xylènes et éventuellement d'éthylbenzène, en présence du catalyseur comprenant une zéolithe préparée selon le procédé de la présente invention.

Le catalyseur préparé selon le procédé de la présente invention est mis en oeuvre dans les réactions d'isomérisation d'une coupe C8 aromatique, comprenant par exemple soit uniquement un mélange de xylène(s), soit uniquement de l'éthylbenzène, soit un mélange de xylène(s) et d'éthylbenzène. Ledit procédé est mis en oeuvre généralement selon les conditions opératoires suivantes :

- une température comprise entre 300°C et 500°C bornes incluses, de préférence entre 320°C et 450°C bornes incluses et de manière encore plus préférée entre 340°C et 430°C bornes incluses,
- une pression partielle d'hydrogène comprise entre 0,3 et 1,5 MPa bornes incluses, de préférence entre 0,4 et 1,2 MPa bornes incluses et de manière encore préférée entre 0,7 et 1,2 MPa bornes incluses,
- une pression totale comprise entre 0,45 et 1,9 MPa bornes incluses, de préférence entre 0,6 et 1,5 MPa bornes incluses,
- une vitesse spatiale d'alimentation, exprimée en kilogramme de charge introduite par kilogramme de catalyseur et par heure, comprise entre 0,25 et $30h^{-1}$ bornes incluses, de préférence entre 1 et $10h^{-1}$ bornes incluses, et de manière encore préférée entre 2 et 6 $h^{-1}$ bornes incluses.

Le catalyseur utilisé dans la présente invention, mis sous forme de billes ou d'extrudés mécaniquement résistants, constitué d'au moins une zéolithe de type structural EUO, par exemple la zéolithe EU-1, obtenue à partir du mode de synthèse décrit dans le présent brevet, d'au moins un liant, d'au moins un métal choisi parmi les éléments du groupe VIII de la classification périodique des éléments, ledit métal étant déposé de préférence sur le liant, présente d'excellentes performances catalytiques en transformations d'hydrocarbures, en termes d'activité, de sélectivité et de stabilité temporelle, comme par exemple en isomérisation des coupes C8 aromatiques, c'està-dire de mélanges constitués de xylènes et éventuellement d'éthylbenzène. Les zéolithes de type EUO utilisées pour l'obtention de ce catalyseur sont obtenues pour des temps de synthèses beaucoup plus courts que les zéolithes de type EUO décrites dans l'art antérieur. Cette méthode de synthèse particulière conduit donc à un gain de coût sur l'élaboration du catalyseur, d'autant plus que les précurseurs utilisés sont moins onéreux que le structurant lui-même, et cela sans dégradation des propriétés catalytiques dudit catalyseur. Par ailleurs, les précurseurs sont moins dangereux que le structurant ce qui permet d'accroître la sécurité lors de la synthèse.

L'invention est illustrée par les exemples suivants.

EXEMPLES 1 à 7: (Comparatif)

Synthèse de zéolithe EU-1 (ou TPZ-3) de rapport Si/Al variable de 15 à 100 avec le bromure d'hexaméthonium (bromure de 1,6 hexaméthylhexaméthylènediammonium) comme structurant organique

Le mélange de synthèse a la composition molaire suivante :

| Exemple | 1 Si/Al=15 | 2 Si/Al=15 | 3 et 4 Si/Al=30 | 5 Si/Al=40 | 6 Si/Al=70 | 7 Si/Al=100 |
|---|---|---|---|---|---|---|
| $SiO_2$ (mol) | 60 | 60 | 60 | 60 | 60 | 60 |
| $Al_2O_3$ (mol) | 2 | 2 | 1 | 0,75 | 0,43 | 0,3 |
| $Na_2O$ (mol) | 10 | 10 | 10 | 10 | 5 | 5 |
| $HxBr_2$ (mol) | 20 | 20 | 20 | 20 | 20 | 20 |
| $H_2O$ (mol) | 2800 | 2800 | 2800 | 2800 | 2800 | 2800 |
| EU-1/$SiO_2$ (g/g)) | 0 | 0,04 | 0,04 | 0,04 | 0,04 | 0,04 |
| $Hx\ Br_2$ = bromure d'hexaméthonium = $Me_3\ N\ (CH_2)_6\ N\ Me_3^{2+}\ (Br^-)_2$ | | | | | | |

On prépare la solution A composée de silicium et de structurant en diluant le bromure d'hexaméthonium (Fluka, 97 %) dans 80 % de l'eau nécessaire à la formation du gel puis en ajoutant le sol de silice colloïdal (Ludox HS40, Dupont, 40 % $SiO_2$). On dissout ensuite l'hydroxyde de sodium solide (Prolabo, 99 %) et l'aluminate de sodium solide (Prolabo, 46 % $Al_2O_3$, 33 % $Na_2O$) dans 10 % de l'eau nécessaire à la formation du gel pour former la solution B. On ajoute la solution B dans la solution A sous agitation puis le restant d'eau (10 %). On mélange jusqu'à homogénéisation et on ajoute les germes de zéolithe EU-1 séchée. On fait réagir le mélange résultant dans un autoclave de 125 ml sous agitation à 180°C sous la pression autogène. Après refroidissement, on filtre le produit et on le lave avec 0,5 litre d'eau déminéralisée puis on le sèche en étuve ventilée à 120°C. Les quantités de réactifs introduites sont reportées dans le tableau suivant :

| Exemple | 1 Si/Al=15 | 2 Si/Al=15 | 3 et 4 Si/Al=30 | 5 Si/Al=40 | 6 Si/Al=70 | 7 Si/Al=100 |
|---|---|---|---|---|---|---|
| Silice colloïdale (g) | 14,50 | 14,50 | 14,52 | 14,53 | 14,61 | 14,61 |
| Aluminate de Sodium (g) | 0,715 | 0,715 | 0,358 | 0,269 | 0,155 | 0,108 |
| Soude (g) | 0,985 | 0,985 | 1,139 | 1,177 | 0,584 | 0,604 |
| $HxBr_2$ (g) | 12,03 | 12,03 | 12,05 | 12,05 | 12,12 | 12,12 |
| Eau (g) | 71,77 | 71,77 | 71,93 | 71,97 | 72,53 | 72,55 |
| Germes EU-1 (g) | 0 | 0,232 | 0,232 | 0,232 | 0,234 | 0,234 |
| $Hx\ Br_2$ = bromure d'hexaméthonium = $Me_3\ N\ (CH_2)_6\ N\ Me_3^{2+}\ (Br^-)_2$ | | | | | | |

Les résultats de diffraction des rayons X et d'analyse chimique sont reportés dans le tableau 1. Les synthèses des exemples 2, 3, 5, 6 et 7 réalisées à 180°C avec le structurant de l'art antérieur, avec des germes de EU-1, conduisent à la zéolithe EU-1 pure (cristallinité de 100 % ± 3) de rapport Si/Al variable entre 15 et 100, de rendement maximal (environ 5 %). L'exemple 1 correspond à une préparation sans germes et nécessite un temps de cristallisation plus long par comparaison avec l'exemple 2 pour produire la zéolithe EU-1 (125 heures contre 96 heures). L'exemple 4 donne majoritairement la zéolithe EU-1 avec un peu de cristobalite du fait d'une durée de cristallisation trop longue par comparaison avec l'exemple 3 (96 heures contre 72 heures).

EXEMPLE 8 à 13 : (comparatif)

Synthèse de zéolithe EU-1 (ou TPZ-3) de rapport Si/Al variable de 15 à 100 avec les précurseurs de l'iodure d'hexaméthonium (iodure de 1,6 hexaméthylhexaméthylènediammonium) comme structurant organique contenant une diamine (1-6 tétraméthylhexaméthylène diamine et iodure de méthyle)

Le mélange de synthèse a la composition molaire suivante:

| Exemple | 8<br>Si/Al=15 | 9<br>Si/Al=15 | 10<br>Si/Al=30 | 11<br>Si/Al=40 | 12<br>Si/Al=70 | 13<br>Si/Al=100 |
|---|---|---|---|---|---|---|
| $SiO_2$ (mol) | 60 | 60 | 60 | 60 | 60 | 60 |
| $Al_2O_3$ (mol) | 2 | 2 | 1 | 0,75 | 0,43 | 0,3 |
| $Na_2O$ (mol) | 10 | 10 | 10 | 10 | 5 | 5 |
| TMHMDA (mol) | 20 | 20 | 20 | 20 | 20 | 20 |
| $CH_3I$ (mol) | 40 | 40 | 40 | 40 | 40 | 40 |
| $H_2O$ (mol) | 2800 | 2800 | 2800 | 2800 | 2800 | 2800 |
| EU-1/$SiO_2$ (g/g) | 0 | 0,04 | 0,04 | 0,04 | 0,04 | 0,04 |
| TMHMDA = 1-6 tétraméthylhexaméthylène diamine = $Me_2 N (CH_2)_6 N Me_2$ | | | | | | |

La préparation est la même que celle décrite dans les exemples 1 à 7 avec en outre le mélange des deux précurseurs au départ, la 1-6 tétraméthylhexaméthylène diamine (Acros, 99 %) et l'iodure de méthyle (Acros, 99 %), dans les 80 % d'eau nécessaire à la formation du gel. Les quantités de réactifs introduites sont reportées dans le tableau suivant :

| Exemple | 8<br>Si/Al=15 | 9<br>Si/Al=15 | 10<br>Si/Al=30 | 11<br>Si/Al=40 | 12<br>Si/Al=70 | 13<br>Si/Al=100 |
|---|---|---|---|---|---|---|
| Silice colloïdale (g) | 14,07 | 14,07 | 14,10 | 14,10 | 14,18 | 14,18 |
| Aluminate de sodium (g) | 0,69 | 0,694 | 0,347 | 0,261 | 0,150 | 0,105 |
| Soude (g) | 0,956 | 0,956 | 1,105 | 1,143 | 0,566 | 0,586 |
| TMHMDA (g) | 5,43 | 5,43 | 5,44 | 5,44 | 5,45 | 5,47 |
| $CH_3I$ (g) | 8,97 | 8,97 | 8,99 | 8,99 | 9,04 | 9,04 |
| Eau (g) | 69,87 | 69,87 | 70,02 | 70,06 | 70,59 | 70,61 |
| Germes EU-1 (g) | 0 | 0,225 | 0,226 | 0,226 | 0,227 | 0,227 |
| TMHMDA = 1-6 tétraméthylhexaméthylène diamine = $Me_2 N (CH_2)_6 N Me_2$ | | | | | | |

Les résultats de diffraction des rayons X et d'analyse chimique sont reportés dans le tableau 2. Les synthèses des exemples 9, 10, 11, 12 et 13 réalisées à 180°C avec le structurant de l'art antérieur, avec des germes de EU-1, conduisent à la zéolithe EU-1 pure (cristallinité de 100 % ± 3) de rapport Si/Al variable entre 15 et 100, de rendement maximal (environ 5 %). L'exemple n°8 correspond à une préparation sans germes et nécessite un temps de cristallisation plus long par comparaison avec l'exemple 9 pour produire la zéolithe EU-1 (122 heures contre 93 heures).

EXEMPLES 14 à 20: (invention)

Synthèse de zéolithe EU-1 (ou TPZ-3) de rapport Si/Al variable entre 15 et 100 avec des précurseurs spécifiques du bromure d'hexaméthonium (bromure de 1,6 hexaméthylhexaméthylènediammonium) comme structurant organique contenant une monoamine (triméthylamine et dibromohexane)

Les mélanges de synthèse ont la composition molaire suivante :

| Exemple | 14<br>Si/Al=15 | 15<br>Si/Al=15 | 16 et 17<br>Si/Al=30 | 18<br>Si/Al=40 | 19<br>Si/Al=60 | 20<br>Si/Al=100 |
|---|---|---|---|---|---|---|
| $SiO_2$ (mol) | 60 | 60 | 60 | 60 | 60 | 60 |
| $Al_2O_3$ (mol) | 2 | 2 | 1 | 0,75 | 0,43 | 0,3 |
| $Na_2O$ (mol) | 10 | 10 | 10 | 10 | 5 | 5 |
| DBH (mol) | 20 | 20 | 20 | 20 | 20 | 20 |
| TMA (mol) | 40 | 40 | 40 | 40 | 40 | 40 |
| $H_2O$ (mol) | 2800 | 2800 | 2800 | 2800 | 2800 | 2800 |
| EU-1/$SiO_2$ (g/g)) | 0 | 0,04 | 0,04 | 0,04 | 0,04 | 0,04 |
| DBH = dibromohexane<br>TMA = triméthylamine | | | | | | |

La préparation est la même que celle décrite dans les exemples 1 à 7 avec en outre le mélange des deux précurseurs au départ, la solution aqueuse de triméthylamine (Acros, 45 %) et le dibromohexane (Acros, 99 %), dans les 80 % d'eau nécessaire à la formation du gel. Les quantités de réactifs introduites sont reportées dans le tableau suivant :

| Exemple | 14<br>Si/Al=15 | 15<br>Si/Al=15 | 16 et 17<br>Si/Al=30 | 18<br>Si/Al=40 | 19<br>Si/Al=60 | 20<br>Si/Al=100 |
|---|---|---|---|---|---|---|
| Silice colloïdale (g) | 14,50 | 14,50 | 14,52 | 14,53 | 14,61 | 14,61 |
| Aluminate de sodium (g) | 0,715 | 0,715 | 0,358 | 0,269 | 0,180 | 0,108 |
| Soude (g) | 0,985 | 0,985 | 1,139 | 1,177 | 0,573 | 0,604 |
| DBH (g) | 8,02 | 8,02 | 8,03 | 8,04 | 8,08 | 8,09 |
| TMA (g) | 8,45 | 8,45 | 8,46 | 8,47 | 8,51 | 8,52 |
| Eau (g) | 67,3 | 67,33 | 67,48 | 67,52 | 68,04 | 68,07 |
| Germes EU-1 (g) | 0 | 0,232 | 0,232 | 0,232 | 0,234 | 0,234 |
| DBH = dibromohexane<br>TMA = triméthylamine | | | | | | |

Les résultats de diffraction des rayons X et d'analyse chimique sont reportés dans le tableau 3. Les synthèses des exemples 15, 16, 18, 19 et 20 réalisées à 180°C avec les précurseurs spécifiques du structurant selon l'invention, avec des germes de EU-1, conduisent à la zéolithe EU-1 pure (cristallinité de 100 % ± 3) de rapport Si/Al variable entre 15 et 100, de rendement maximal (environ 5 %). L'exemple 14 correspond à une préparation sans germes et nécessite un temps de cristallisation plus long par comparaison avec l'exemple 15 pour produire la zéolithe EU-1 (110 heures contre 85 heures). L'exemple 17 donne majoritairement la zéolithe EU-1 avec un peu de cristoballite du fait d'une durée de cristallisation plus longue par comparaison avec l'exemple 16 (82 heures contre 65 heures).

Tableau 1:

| Synthèses des zéolithes EU-1 avec le structurant organique selon l'art antérieur | | | | | | | |
|---|---|---|---|---|---|---|---|
| n° exemple | **1** | **2** | **3** | **4** | **5** | **6** | **7** |
| **Gel** | | | | | | | |
| formulation | 60 $SiO_2$ - a $Al_2O_3$ - b $Na_2O$ - 20 $HxBr_2$ - 2800 $H_2O$ - 0-4 % EU-1 | | | | | | |
| **Si/Al=30/a (mol/mol)** | 15 | 15 | 30 | 30 | 40 | 70 | 100 |
| **alcalinité b (mol)** | 10 | 10 | 10 | 10 | 10 | 5 | 5 |

Tableau 1:   (suite)

| Synthèses des zéolithes EU-1 avec le structurant organique selon l'art antérieur | | | | | | |
|---|---|---|---|---|---|---|
| **n° exemple** | **1** | **2** | **3** | **4** | **5** | **6** | **7** |
| **Gel** | | | | | | |
| **germes** | Non | oui | oui | oui | Oui | oui | oui |
| **Cristallisation** | | | | | | |
| **température (°C)** | 180 | | | | | | |
| **temps (h)** | 125 | 96 | 72 | 96 | 72 | 72 | 60 |
| **Solide** | | | | | | |
| **phase (DRX)** | 100% EU-1 | 101 % EU-1 | 100% EU-1 | 90 % EU-1 + 10 % CRISTO | 98 % EU-1 | 99 % EU-1 | 102 % EU-1 |
| **Si/Al (FX)** | 14,2 | 13,9 | 26,6 | nd | 36,9 | 68,6 | 90,7 |
| **rendement en solide (%)** | 5,1 | 5,8 | 5,2 | 5,3 | 5,1 | 5.6 | 5,2 |

CRISTO : cristobalite

Hx Br$_2$ = bromure d'hexaméthonium = Me$_3$ N (CH$_2$)$_6$ N Me$_3$$^{2+}$ (Br$^-$)$_2$

DRX : diffraction des rayons X avec comme référence l'exemple 1

FX : fluorescence X

Tableau 2 :

| Synthèses des zéolithes EU-1 avec les précurseurs de structurant organique selon l'art antérieur | | | | | | |
|---|---|---|---|---|---|---|
| **n° exemple** | **8** | **9** | **10** | **11** | **12** | **13** |
| **Gel** | | | | | | |
| **formulation** | 60 SiO$_2$ - a Al$_2$O$_3$ - b Na$_2$O - 20 TMHDA - 40 CH$_3$I - 2800 H$_2$O - 4 % EU-1 | | | | | |
| **Si/Al=30/a (mol/mol)** | 15 | 15 | 30 | 40 | 70 | 100 |
| **alcalinité b (mol)** | 10 | 10 | 10 | 10 | 5 | 5 |
| **Cristallisation** | | | | | | |
| **température (°C)** | 180 | | | | | |
| **temps (h)** | 122 | 93 | 70 | 70 | 70 | 60 |
| **Solide** | | | | | | |
| **phase (DRX)** | 100 % EU-1 | 99 % EU-1 | 98 % EU-1 | 101 % EU-1 | 100 % EU-1 | 98 % EU-1 |
| **Si/Al (FX)** | 14,2 | 13,8 | 13,2 | 13,7 | 14,3 | 13,5 |
| **rendement en solide (%)** | 5,0 | 5,2 | 4,8 | 5,6 | 5,4 | 5,1 |

TMHMDA = 1;6 tétraméthylhexaméthylène diamine = Me$_2$ N (CH$_2$)$_6$ N Me$_2$

DRX : diffraction des rayons X avec comme référence l'exemple 8

FX : fluorescence X

Tableau 3 :

| Synthèses des zéolithes EU-1 avec les précurseurs du structurant organique selon l'invention | | | | | | | |
|---|---|---|---|---|---|---|---|
| n° exemple | 14 | 15 | 16 | 17 | 18 | 19 | 20 |
| Gel | | | | | | | |
| formulation | 60 SiO$_2$ - a Al$_2$O$_3$ - b Na$_2$O - 20 DBrH - 40 TMA - 2800 H$_2$O - 0-4 % EU-1 | | | | | | |
| Si/Al=30/a | 15 | 15 | 30 | 30 | 40 | 60 | 100 |
| alcalinité b (mol) | 10 | 10 | 10 | 10 | 10 | 5 | 5 |
| germes | non | oui | oui | oui | Oui | oui | oui |
| Cristallisation | | | | | | | |
| température (°C) | 180 | | | | | | |
| temps (h) | 110 | 85 | 65 | 82 | 65 | 65 | 53 |
| Solide | | | | | | | |
| phase (DRX) | 100% EU-1 | 102 % EU-1 | 101 % EU-1 | 90 % EU-1 + 10 % CRISTO | 99 % EU-1 | 100% EU-1 | 98% EU-1 |
| Si/Al (FX) | 14,4 | 14,2 | 25,4 | nd | 34,6 | 57,6 | 92,3 |
| rendement en solide (%) | 5,5 | 5,0 | 5,5 | 5,2 | 5,6 | 5,7 | 5,8 |
| CRISTO : cristobalite<br>DBH = dibromohexane<br>TMA = triméthylamine<br>DRX : diffraction des rayons X avec comme référence l'exemple 14<br>FX : fluorescence X | | | | | | | |

Bilan des synthèses de zéolithe EU-1

Les données des tableaux 1, 2 et 3 montrent que les zéolithes EU-1 synthétisées à l'aide de précurseurs spécifiques du structurant organique contenant une monoamine sont cristallisées en un temps plus court que les zéolithes EU-1 synthétisées par une méthode selon l'art antérieur, ce qui permet un gain de coût. De plus, lors de la mise en oeuvre de l'ajout de germes de zéolithe lors de la préparation, on améliore encore les performances en terme de temps de cristallisation.

***Exemple 21 :*** Préparation du catalyseur A non conforme à l'invention, contenant la zéolithe EU-1 de l'exemple 1 et 0,3 % poids de platine.

La matière première utilisée est la zéolithe EU-1 brute de synthèse référencée de l'exemple 1, comprenant le structurant organique, du silicium et de l'aluminium, possédant un rapport atomique Si/Al global égal à 14,2 , une teneur pondérale en sodium par rapport au poids en zéolithe EU-1 sèche d'environ 1,5%.

Cette zéolithe EU-1 subit tout d'abord une calcination dite sèche à 550°C sous flux d'air durant 6 heures. Puis le solide obtenu est soumis à trois échanges ioniques dans une solution de NH$_4$NO$_3$ 10N, à environ 100°C pendant 4 heures pour chaque échange.

A l'issue de ces traitements, la zéolithe EU-1 sous forme NH$_4$ a un rapport Si/Al atomique global égal à 18,8 , une teneur pondérale en sodium par rapport au poids de zéolithe EU-1 sèche de 50 ppm.

La zéolithe EU-1 est ensuite mise en forme par extrusion avec un gel d'alumine de manière à obtenir, après séchage et calcination sous air sec, un support S1 constitué d'extrudés de 1,4 mm de diamètre, qui contient en poids 10 % de zéolithe EU-1 sous forme H et 90 % d'alumine.

Le support S1 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur A ainsi obtenu contient en poids, 10,0 % de zéolithe EU-1 forme hydrogène, 89,7 % d'alumine et 0,3 % de platine.

*Exemple 22 :* Préparation du catalyseur B non conforme à l'invention, contenant la zéolithe EU-1 de l'exemple 2 et 0,3 % poids de platine.

La matière première utilisée est la zéolithe EU-1 brute de synthèse de l'exemple 2, comprenant le structurant organique, du silicium et de l'aluminium, possédant un rapport atomique Si/Al global égal à 13,9 , une teneur pondérale en sodium par rapport au poids en zéolithe EU-1 sèche d'environ 1,5 %.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/Al atomique global égal à 18,5 , une teneur pondérale en sodium par rapport au poids de zéolithe EU-1 sèche de 45 ppm.

La zéolithe EU-1 est ensuite mise en forme par extrusion avec un gel d'alumine de manière à obtenir, après séchage et calcination sous air sec, un support S2 constitué d'extrudés de 1,4 mm de diamètre, qui contient en poids 10 % de zéolithe EU-1 sous forme H et 90 % d'alumine.

Le support S2 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur B ainsi obtenu contient en poids, 10,0 % de zéolithe EU-1 forme hydrogène, 89,7 % d'alumine et 0,3 % de platine.

*Exemple 23 :* Préparation du catalyseur C conforme à l'invention, contenant la zéolithe EU-1 de l'exemple 14 et 0,3 % poids de platine.

La matière première utilisée est la zéolithe EU-1 brute de synthèse de l'exemple 14, comprenant le structurant organique, du silicium et de l'aluminium, possédant un rapport atomique Si/Al global égal à 14,4 , une teneur pondérale en sodium par rapport au poids en zéolithe EU-1 sèche d'environ 1,5.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/Al atomique global égal à 18,7 , une teneur pondérale en sodium par rapport au poids de zéolithe EU-1 sèche de 30 ppm.

La zéolithe EU-1 est ensuite mise en forme par extrusion avec un gel d'alumine de manière à obtenir, après séchage et calcination sous air sec, un support S3 constitué d'extrudés de 1,4 mm de diamètre, qui contient en poids 10 % de zéolithe EU-1 sous forme H et 90 % d'alumine.

Le support S3 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur C ainsi obtenu contient en poids, 10,0 % de zéolithe EU-1 forme hydrogène, 89,7 % d'alumine et 0,3 % de platine.

*Exemple 24 :* Préparation du catalyseur D conforme à l'invention, contenant la zéolithe EU-1 de l'exemple 15 et 0,3 % poids de platine.

La matière première utilisée est la zéolithe EU-1 brute de synthèse de l'exemple 15, comprenant le structurant organique, du silicium et de l'aluminium, possédant un rapport atomique Si/Al global égal à 14,2 , une teneur pondérale en sodium par rapport au poids en zéolithe EU-1 sèche d'environ 1,5.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/Al atomique global égal à 18,6 , une teneur pondérale en sodium par rapport au poids de zéolithe EU-1 sèche de 40 ppm.

La zéolithe EU-1 est ensuite mise en forme par extrusion avec un gel d'alumine de manière à obtenir, après séchage et calcination sous air sec, un support S4 constitué d'extrudés de 1,4 mm de diamètre, qui contient en poids 10 % de zéolithe EU-1 sous forme H et 90 % d'alumine.

Le support S4 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur D ainsi obtenu contient en poids, 10,0 % de zéolithe EU-1 forme hydrogène, 89,7 % d'alumine et 0,3 % de platine.

***Exemple 25 :*** Préparation du catalyseur E non conforme à l'invention, contenant la zéolithe EU-1 de l'exemple 8 et 0,3 % poids de platine.

La matière première utilisée est la zéolithe EU-1 brute de synthèse de l'exemple 8, comprenant le structurant organique, du silicium et de l'aluminium, possédant un rapport atomique Si/Al global égal à 14,2 , une teneur pondérale en sodium par rapport au poids en zéolithe EU-1 sèche d'environ 1,5.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/Al atomique global égal à 18,7 , une teneur pondérale en sodium par rapport au poids de zéolithe EU-1 sèche de 40 ppm.

La zéolithe EU-1 est ensuite mise en forme par extrusion avec un gel d'alumine de manière à obtenir, après séchage et calcination sous air sec, un support S5 constitué d'extrudés de 1,4 mm de diamètre, qui contient en poids 10 % de zéolithe EU-1 sous forme H et 90 % d'alumine.

Le support S5 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur E ainsi obtenu contient en poids, 10,0 % de zéolithe EU-1 forme hydrogène, 89,7 % d'alumine et 0,3 % de platine.

***Exemple 26 :*** Préparation du catalyseur F non conforme à l'invention, contenant la zéolithe EU-1 de l'exemple 9 et 0,3 % poids de platine.

La matière première utilisée est la zéolithe EU-1 brute de synthèse de l'exemple 9, comprenant le structurant organique, du silicium et de l'aluminium, possédant un rapport atomique Si/Al global égal à 13,8, une teneur pondérale en sodium par rapport au poids en zéolithe EU-1 sèche d'environ 1,5.

Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange.

A l'issue de ces traitements, la zéolithe EU-1 sous forme $NH_4$ a un rapport Si/Al atomique global égal à 18,4 , une teneur pondérale en sodium par rapport au poids de zéolithe EU-1 sèche de 45 ppm.

La zéolithe EU-1 est ensuite mise en forme par extrusion avec un gel d'alumine de manière à obtenir, après séchage et calcination sous air sec, un support S6 constitué d'extrudés de 1,4 mm de diamètre, qui contient en poids 10 % de zéolithe EU-1 sous forme H et 90 % d'alumine.

Le support S6 ainsi obtenu est soumis à un échange anionique avec de l'acide hexachloroplatinique en présence d'un agent compétiteur (acide chlorhydrique), de manière à déposer 0,3 % poids de platine par rapport au catalyseur. Le solide humide est ensuite séché à 120°C pendant 12 heures et calciné sous un débit d'air sec à la température de 500°C pendant une heure.

Le catalyseur F ainsi obtenu contient en poids, 10,0 % de zéolithe EU-1 forme hydrogène, 89,7 % d'alumine et 0,3 % de platine.

***Exemple 27 :*** Evaluation des propriétés catalytiques des catalyseurs A, B, C, D, E et F en isomérisation d'une coupe C8 aromatique.

Les performances des catalyseurs A, B, C, D, E et F ont été évaluées dans l'isomérisation d'une coupe C8 aromatique contenant principalement du méta-xylène, de l'ortho-xylène et de l'éthylbenzène. Les conditions opératoires sont les suivantes :

- température : 390°C,
- pression totale : 15 bar, (1 bar = 0,1 MPa)
- pression partielle d'hydrogène : 12 bar.

Les catalyseurs sont préalablement traités avec une charge contenant du disulfure de diméthyle (DMDS) en présence d'hydrogène, avec une concentration telle que le rapport atomique soufre/métal soit de 1,5. Le catalyseur est ensuite maintenu pendant 3 heures à 400°C sous débit d'hydrogène puis la charge est injectée.

Les catalyseurs sont testés en laboratoire, par 5 g et sans recyclage d'hydrogène.

Les catalyseurs ont été comparés en terme d'activité (par les approches à l'équilibre du para-xylène et de l'éthylbenzène, et par la conversion de l'éthylbenzène), et en terme de sélectivité par les pertes nettes à iso-approche à l'équilibre du para-xylène.

Les réactions parasites conduisent à trois types de pertes: les pertes vers les paraffines résultant essentiellement de réactions d'ouverture de cycles naphténiques suivies de craquage, les pertes vers les aromatiques formés par réactions de dismutation et de transalkylation des aromatiques à 8 atomes de carbone (AC8), et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes) en prenant une base 100 pour les pertes nettes du catalyseur A non conforme à l'invention.

Pour le calcul des approches à l'équilibre (AEQ), les concentrations en paraxylènes (%pX) sont exprimées, par rapport aux trois isomères xylènes.

L'approche à l'équilibre (AEQ) est définie de la manière suivante :

$$pX\ AEQ\ (\%) = 100\ x\ (\%pX_{effluent} - \%pX_{charge}) / (\%pX_{équilibre} - \%pX_{charge})$$

Les pertes par craquage (P1) sont des pertes en AC8 sous forme de paraffines (PAR) de C1 à C8 :

$$P1(\%poids) = 100\ X\ [(\%PAR_{effluent}xpoids\ d'effluent) - (\%PAR_{charge}xpoids\ de\ charge)] / (\%AC8_{charge}xpoids\ de\ charge)$$

Les pertes par dismutation/transalkylation (P2) sont des pertes en AC8 sous forme de naphtènes autres que N8, de toluène, de benzène et de C9+ aromatiques (OAN) :

$$P2(\%poids) = 100\ X\ [(\%OAN_{effluent}xpoids\ d'effluent) - (\%OAN_{charge}xpoids\ de\ charge)] / (\%AC8_{charge}xpoids\ de\ charge)$$

La somme des pertes P1 et P2 représente les pertes nettes.

Les données présentées dans le tableau 4 ont été obtenues à iso-conditions expérimentales.

Tableau 4

| Catalyseurs | A (comp.) | B (comp.) | C (inv.) | D (inv.) | E (comp.) | F (comp.) |
|---|---|---|---|---|---|---|
| pX AEQ (%) | 98,1 | 98,3 | 98,0 | 97,8 | 98,3 | 98,2 |
| conversion EB (%) | 59,6 | 58,8 | 59,5 | 59,3 | 59,4 | 59,6 |

On constate d'après les résultats du tableau 4 que les catalyseurs C et D conformes à l'invention conduisent à des résultats comparables à ceux obtenus avec les catalyseurs A, B, E et F non conformes.

Par ailleurs, ces catalyseurs ont été comparés à pX AEQ plus faible (environ 95,5%) en faisant varier les débits massiques de charge. Ces résultats sont présentés dans le tableau 5.

Tableau 5

| Catalyseurs | A | B | C | D | E | F |
|---|---|---|---|---|---|---|
| pX AEQ (%) | 95,5 | 95,2 | 95,7 | 95,1 | 95,5 | 95,3 |

Tableau 5   (suite)

| Catalyseurs | A | B | C | D | E | F |
|---|---|---|---|---|---|---|
| pertes nettes (% poids) | 4,9 | 4,7 | 5,0 | 4,6 | 4,8 | 4,9 |

A iso pX AEQ plus faible, le tableau 5 confirme les résultats présentés dans le tableau 4 et montre que les catalyseurs sont tous aussi sélectifs.

L'activité et la sélectivité obtenues lors de l'utilisation des catalyseurs C et D, à base de zéolithes de structure EUO, obtenues en utilisant des précurseurs du structurant organique en isomérisation d'une coupe C8 aromatique sont donc comparables à celles des catalyseurs contenant des zéolithes de structure EUO de rapport Si/Al proche et obtenue selon un mode de synthèse au cours duquel le structurant organique lui-même est utilisé comme décrit dans l'art antérieur. Ce résultat est également vérifié dans le cas de l'utilisation de précurseurs contenant une diamine.

Enfin, les catalyseurs A, B, C, D, E et F ont été comparés en terme de stabilité temporelle toujours dans les conditions expérimentales décrites au début de cet exemple et sur une durée de 400 heures.

La stabilité est évaluée à partir de l'évolution de la conversion de l'éthylbenzène. Les résultats sont présentés dans le tableau 6.

Tableau 6

| Catalyseur | A | B | C | D | E | F |
|---|---|---|---|---|---|---|
| conversion EB (%) à t=36 h | 59,6 | 59,8 | 59,5 | 59,3 | 59,4 | 59,6 |
| conversion EB (%) à t=400 h | 57,6 | 57,7 | 57,3 | 57,4 | 57,5 | 57,6 |
| chute de conversion EB (%) | 3,3 | 3,5 | 3,6 | 3,2 | 3,2 | 3,4 |

On constate que la désactivation des six catalyseurs A, B, C, D, E et F sur 400 heures est comparable.

Les propriétés catalytiques (activité, sélectivité et stabilité) des six catalyseurs sont comparables. L'introduction de ce nouveau mode de synthèse de la zéolithe de type structural EUO dans la préparation de ce catalyseur d'isomérisation des coupes C8 aromatiques est donc d'un apport tout à fait notable en terme de coût puisque les précurseurs sont moins onéreux et que le temps de synthèse de la zéolithe est réduit et tout en conservant ses propriétés catalytiques. De plus, l'utilisation de ces précurseurs du structurant contenant une monoamine limite les risques liés à la toxicité.

**Revendications**

1.   Procédé de synthèse d'un matériau zéolithique de typé structural EUO comprenant au moins un élément X choisi parmi le silicium et le germanium et au moins un élément T choisi parmi l'aluminium, le fer, le gallium, le bore, le titane, le vanadium, le zirconium, le molybdène, l'arsenic, l'antimoine, le chrome et le manganèse, ledit procédé comprenant le mélange en milieu aqueux d'au moins une source d'au moins un élément X, d'au moins une source d'au moins un élément T, et d'au moins deux précurseurs d'un structurant comprenant un dérivé alkylé de polyméthylène $\alpha$-$\omega$ diammonium, l'un des précurseurs étant choisi parmi les monoamines et l'autre précurseur étant du type F-R-F' où F et F' sont des groupements partants identiques ou différents.

2.   Procédé selon la revendication 1 tel que le dérivé alkylé de polyméthylène $\alpha$-$\omega$ diammonium a la formule suivante : $R_1 R_2 R_3 N^+ (CH_2)_n N^+ R_4 R_5 R_6$ n étant compris entre 3 et 14 et $R_1$ à $R_6$, identiques ou différents, pouvant représenter des radicaux alkyles ou hydroxyalkyles ayant de 1 à 8 atomes de carbone, jusqu'à cinq radicaux $R_1$ à $R_6$ pouvant être de l'hydrogène.

3.   Procédé selon l'une des revendications 1 à 2 dans lequel le dérivé alkylé de polyméthylène $\alpha$-$\omega$ diammonium est un composé d'hexaméthylènediammonium alkylé.

4.   Procédé selon l'une des revendications 1 à 3, dans lequel le dérivé alkylé de polyméthylène $\alpha$-$\omega$ diammonium est un sel de 1,6-N,N,N',N',N'-hexaméthylhexaméthylènediammonium.

5.   Procédé selon l'une des revendications 1 à 4, dans lequel le précurseur du dérivé alkylé de polyméthylène $\alpha$-$\omega$ diammonium choisi parmi les monoamines est une trialkylamine.

17

**EP 0 999 183 B1**

6. Procédé selon l'une des revendications 1 à 5, dans lequel le précurseur choisi parmi les monoamines est la triméthylamine (TMA).

7. Procédé selon l'une des revendication 1 à 6 dans lequel le précurseur du type F-R-F' est choisi parmi les dihalogénures d'alcane et les alcanediols.

8. Procédé selon la revendication 7 dans lequel le précurseur est le dibromohexane.

9. Procédé selon l'une des revendications 1 à 8 dans lequel on ajoute au mélange réactionnel au moins un germe d'au moins une zéolithe.

10. Procédé selon l'une des revendications 1 à 9 dans lequel on ajoute au mélange au moins un sel P.

11. Procédé selon l'une des revendications 1 à 10 dans lequel le mélange réactionnel a la composition molaire suivante, exprimée sous forme d'oxydes :

| $XO_2/T_2O_3$ (mol/mol) | au moins 10 |
|---|---|
| $Q/XO_2$ (mol/mol) | 0,002 à 2,0 |
| $OH^-/XO_2$ (mol/mol) | 0,002 à 2,0 |
| $Q/(M^++Q)$ (mol/mol) | 0,1 à 1 |
| $H_2O/XO_2$ (mol/mol) | 1 à 500 |
| $P/XO_2$ (mol/mol) | 0 à 5 |
| $S/XO_2$ (g/g) | 0 à 0,1 |

où $M^+$ représente un métal alcalin ou l'ammonium et Q représente le dérivé alkylé de polyméthylène $\alpha$-$\omega$ diam-monium, introduit à l'aide de précurseurs comprenant une monoamine.

12. Procédé selon l'une des revendications 1 à 11 dans lequel X est le silicium et T est l'aluminium.

13. Procédé selon l'une des revendications 1 à 12 dans lequel on introduit les précurseurs du dérivé alkylé de poly-méthylène $\alpha$-$\omega$ diammonium à n'importe quel moment de la synthèse.

14. Procédé selon l'une des revendications 1 à 13 dans lequel on introduit les précurseurs en solution avant l'addition de l'élément T et de l'élément X.

**Patentansprüche**

1. Verfahren zur Synthese eines zeolithischen Materials vom Strukturtyp EUO, umfassend wenigstens ein Elements X, gewählt unter Silizium und Germanium, und wenigstens ein Elements T, gewählt unter Aluminium, Eisen, Gallium, Bor, Titan, Vanadium, Zirkonium, Molybdän, Arsen, Antimon, Chrom und Mangan, wobei das Verfahren umfasst, das Mischen im wässrigen Milieu von wenigstens einer Quelle wenigstens eines Elements X; wenigstens einer Quelle wenigstens eines Elements T und wenigstens zwei Vorläufern eines Strukturbildners, welcher ein alkyliertes Derivat von Diammonium-$\alpha$-$\omega$-Polymethylen umfasst, wobei einer der Vorläufer gewählt ist unter den Monoaminen und der andere Vorläufer vom Typ F-R-F' ist, wobei F und F' identische oder unterschiedliche Ausgangsgruppen sind.

2. Verfahren nach Anspruch 1, derart dass das alkylierte Derivat von Diammonium-$\alpha$-$\omega$-Polymethylen folgende For-mel hat: $R_1R_2R_3 N^+ (CH_2)_n N^+ R_4R_5R_6$, wobei n zwischen 3 und 14 liegt und $R_1$ bis $R_6$ identisch oder verschieden Alkyl- oder Hydroxyaklylradikale mit 1 bis 8 Kohlenstoffatomen darstellen können, wobei bis zu fünf Radikale $R_1$ bis $R_6$ Wasserstoff sein können.

3. Verfahren nach einem der Ansprüche 1 bis 2, bei dem das alkylierte Derivat von Diammonium-$\alpha$-$\omega$-Polymethylen eine alkylierte Diammonium-Hexamethylenverbindung ist.

4. Verfahren nach einem der Ansprüche 1 bis 3, bei dem das alkylierte Derivat von Diammonium-$\alpha$-$\omega$-Polymethylen ein Salz vom 1,6-N,N,N',N',N'-Diammonium-Hexamethylhexamethylen ist.

**5.** Verfahren nach einem der Ansprüche 1 bis 4, bei dem der Vorläufer des alkylierten Derivats von Diammonium-$\alpha$-$\omega$-Polymethylen, gewählt unter den Monoaminen, ein Trialkylamin ist.

**6.** Verfahren nach einem der Ansprüche 1 bis 5, bei dem der Vorläufer gewählt unter den Monoaminen, Trimethylamin (TMA) ist.

**7.** Verfahren nach einem der Ansprüche 1 bis 6, bei dem der Vorläufer vom Typ F-R-F' gewählt ist unter den Alkandihalogeniden oder den Alkandiolen.

**8.** Verfahren nach Anspruch 7, bei dem der Vorläufer Dibromohexan ist.

**9.** Verfahren nach einem der Ansprüche 1 bis 8, bei dem man zur Reaktionsmischung wenigstens einen Keim wenigstens eines Zeolithen hinzugibt.

**10.** Verfahren nach einem der Ansprüche 1 bis 9, bei dem man zum Gemisch wenigstens ein Salz P hinzugibt.

**11.** Verfahren nach einem der Ansprüche 1 bis 10, bei dem die Reaktionsmischung die folgende molare Zusammensetzung, ausgedrückt in Oxydform hat:

| | |
|---|---|
| $XO_2$/ $T_2 O_3$. (mol/mol) | wenigstens 10 |
| $Q/XO_2$ (mol/mol) | 0,002 bis 2,0 |
| $OH/XO_2$ (mol/mol) | 0,002 bis 2,0 |
| $Q/(M^+ + Q)$ (mol/mol) | 0,1 bis 1,0 |
| $H_2O/XO_2$ (mol/mol) | 1 bis 500 |
| $P/XO_2$ (mol/mol) | 0 bis 5 |
| $S/XO_2$ (g/g) | 0 bis 0,1 |

wobei $M^+$ ein Alkalimetall oder Ammonium darstellt und Q ein alkyliertes Derivat von Diammonium-$\alpha$-$\omega$-Polymethylen, eingeführt mit Hilfe von Vorläufern, welche ein Monoamin umfassen, darstellt.

**12.** Verfahren nach einem der Ansprüche 1 bis 11, bei dem X Silizium und T Aluminium ist.

**13.** Verfahren nach einem der Ansprüche 1 bis 12, bei dem man die Vorläufer des alkylierten Derivats von Diammonium-$\alpha$-$\omega$-Polymethylen zu irgendeinem Zeitpunkt der Synthese einführt.

**14.** Verfahren nach einem der Ansprüche 1 bis 13, bei dem man die Vorläufer in Lösung vor Zugabe des Elements T und des Elements X einführt.

**Claims**

**1.** A process for synthesising a zeolitic material with structure type EUO comprising at least one element X selected from silicon and germanium and at least one element T selected from aluminium, iron, gallium, boron, titanium, vanadium, zirconium, molybdenum, arsenic, antimony, chromium and manganese, comprising reacting an aqueous mixture of at least one source of at least one element X, at least one source of at least one element T and at least two precursors of a structuring agent comprising an alkylated polymethylene $\alpha$-$\omega$ diammonium derivative, one of the precursors being selected from monoamines and the other one being of F-R-F' type where F and F' are identical or different starting groups.

**2.** A process according to claim 1, in which the alkylated polymethylene $\alpha$-$\omega$ diammonium derivative has the following formula: $R_1R_2R_3N+(CH_2)_nN^+ R_4R_5R_6$, where n is in the range 3 to 14 and $R_1$ to $R_6$, which may be identical or different, can represent alkyl or hydroxyalkyl radicals containing 1 to 8 carbon atoms; up to five $R_1$ to $R_6$ radicals possibly being hydrogen.

**3.** A process according to claim 1 or claim 2, in which the alkylated polymethylene $\alpha$-$\omega$ diammonium derivative is an alkylated hexamethylenediammonium compound.

4. A process according to any one of claims 1 to 3, in which the alkylated polymethylene $\alpha$-$\omega$ diammonium derivative is a 1,6-N,N,N,N',N',N'-hexamethylhexamethylenediammonium salt.

5. A process according to any one of claims 1 to 4, in which the alkylated polymethylene $\alpha$-$\omega$ diammonium derivative selected from monoamines is a trialkylamine.

6. A process according to any one of claims 1 to 5, in which the precursor selected from monoamines is trimethylamine (TMA).

7. A process according to any one of claims 1 to 6, in which the precusor of F-R-F' type is selected from alkanediols and alkane dihalides.

8. A process according to claim 7, in which the precursor is dibromohexane.

9. A process according to any one of claims 1 to 8, in which at least one seed of at least one zeolite is added to the reaction mixture.

10. A process according to any one of claims 1 to 9, in which at least one salt P is added to the mixture.

11. A process according to any one of claims 1 to 10, in which the reaction mixture has the following molar composition, expressed in the oxide form:

| | |
|---|---|
| $XO_2/T_2O_3$ (mol/mol) | at least 10 |
| $Q/XO_2$ (mol/mol) | 0.002 to 2.0 |
| $OH^-/XO_2$ (mol/mol) | 0.002 to 2.0 |
| $Q/(M^+ + Q)$ (mol/mol) | 0.1 to 1 |
| $H_2O/XO_2$ (mol/mol) | 1 to 500 |
| $P/XO_2$ (mol/mol) | 0 to 5 |
| $S/XO_2$ (g/g) | 0 to 0.1 |

where $M^+$ represents an alkali metal or ammonium and Q represents the alkylated polymethylene $\alpha$-$\omega$ diammonium derivative, introduced by means of precursors comprising a monoamine.

12. A process according to any one of claims 1 to 11, in which X is silicon and T is aluminium.

13. A process according to any one of claims 1 to 12, in which the precursors of the alkylated polymethylene $\alpha$-$\omega$ diammonium derivative are introduced at any point in the synthesis.

14. A process according to any one of claims 1 to 13, in which the precursors are introduced in solution before adding the element T and element X.